# EUROPEAN PATENT SPECIFICATION

(11) **EP 2 488 569 B1**
(45) Date of publication and mention of the grant of the patent: **07.01.2015**
(21) Application number: 10760975.2
(22) Date of filing: 21.09.2010
(51) Int. Cl.: C08G 77/04, C08G 77/28, A61K 8/89, C07F 7/08, C08G 77/38

(54) **A SILICONE COMPOUND**
SILIKONVERBINDUNG
COMPOSÉ SILICONE

(30) Priority: 12.10.2009 IN MU23622009
(43) Date of publication of application: 22.08.2012
(73) Proprietor: Unilever N.V., 3013 AL Rotterdam (NL); Unilever PLC, London EC4Y 0DY (GB); Hindustan Unilever Limited, Mumbai 400 099 (IN)
(72) Inventor: VAIDYA, Ashish, Anant, Bangalore 560 066 (IN); GHOSH, Nilmoni, Bangalore 560 066 (IN); KUNJUPILLAI, Balu, Bangalore 560 066 (IN)
(74) Representative: Corsten, Michael Allan
(86) International application number: PCT/EP2010/063890
(87) International publication number: WO 2011/045149

(56) References cited:
- EP-A1- 0 842 938
- US-A1- 2009 209 743

## Description

### TECHNICAL FIELD

The present invention relates to silicone compounds and personal care compositions comprising the same which have photoprotective properties.

### BACKGROUND OF THE INVENTION

Solar radiation includes about 5% ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer. Scientific studies have indicated that exposure to UV-A and UV-B radiation for short period causes reddening of the skin and localized irritation, whereas continued and prolonged exposure can lead to sunburn, melanoma and formation of wrinkles. It is also reported that UV radiation causes significant damage to hair. Therefore it is desirable to protect the skin and other keratinous substrates of the human body from the harmful effects of both UV-A and UV-B radiation. Further, it is also essential that the body is protected against the damaging effects of the visible spectrum of light. The visible part of light ranges from 400 to 800 nm. It is believed that continued exposure to visible light is responsible for damaging effects on skin like erythema, pigmentation, thermal damage and free radical production (Bassel et al, Effects of Visible Light on the Skin, Photochemistry and Photobiology Volume 84 Issue 2, Pages 450-462, 2008). Hence in addition to protection from UV rays it is also necessary to get broader spectrum protection which includes visible light. Personal care products such as creams and lotions are used to mitigate these effects and/or to prevent further damage. For this purpose, sunscreens or sun block agents are generally added therein to protect the skin from the harmful effects of UV radiation.

Organic sunscreens absorb a large fraction of the incident UV radiation, thereby preventing the radiation from coming in contact with the surface of the skin. They have UV absorbing sites, called chromophores, which are primarily responsible for their activity. Some sunscreens absorb UV-A radiation while some absorb UV-B radiation.

Conventional UV-A and UV-B sunscreens have problems associated with relatively low photostability and harmful photodegradation byproducts when used in topical products. One such approach is attaching both UV-A and UV-B moieties to a silicone backbone to provide sunscreen protection is disclosed in a co-pending Indian Patent Application number 2084/MUM/2006.

Further, EP0842938 (L'Oreal, 1999) discloses flavone substituted silicone compounds. US2009/0209743 (Wacker) discloses coloured organopolysiloxanes which are flavone derivatised. Both of the above publications disclose derivatisation of straight chain silicone polymer and there is no mention of crosslinking through a spacer group.

Not many compositions have been disclosed for providing protection against visible radiation, in addition to UV radiation, particularly as the recent evidence suggests that radiation in the visible wavelength can also cause significant skin damage (Bassel et al, Effects of Visible Light on the Skin, Photochemistry and Photobiology Volume 84 Issue 2, Pages 450 - 462, 2008).

Compounds like curcumin that provide some protection against visible radiation have been included in cosmetic compositions. However, compounds such as curcumin suffer from the problem of relatively low photostability and harmful degradation products.

On the other hand, thickeners such DC9040 (Dow Corning) can be used along with compounds that provide photoprotection against visible radiation to provide thickening, but the resulting compositions do not spread uniformly.

The present inventors have been working on the problem of providing a sunscreen that gives broad spectrum protection against both UV radiation and visible radiation. They found to their surprise that when certain moieties generally occurring in nature e.g. curcuminoids, polyphenol or flavonoids having UV-visible absorption activity are attached to crosslinked silicone polymers they provide not only the desired photoprotection but also excellent spreadability on skin and other human keratinous substrates that is essential for providing enhanced photoprotection. Further, the new polymer thus synthesized was highly amenable for further derivatisation with hydrophilic groups that enabled self-emulsification of the polymer in topical products. Further, the compound of the present invention gives enhanced sun protection when incorporated in sunscreen compositions comprising conventional UVA and UVB sunscreens.

It is therefore one of the objects of the present invention to overcome or ameliorate at least one of the disadvantages of the prior art or to provide a useful alternative.

Another object of the present invention is to provide compounds that give relatively better protection against UV and visible radiation and yet have relatively more photostability.

Yet another object of the present invention is to provide compounds that give enhanced photoprotection against UV and visible radiation while having a self-emulsifying property to enable incorporation in personal care compositions without the need for additional emulsifiers.

Yet another object of the present invention is to provide for a personal care sunscreen composition that spreads optimally on the substrate to give the desired photoprotection.

### SUMMARY OF THE INVENTION

According to one aspect of the invention there is provided a silicone compound of the general formula: 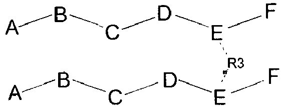 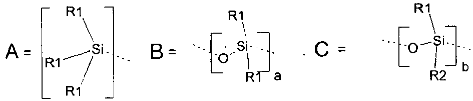 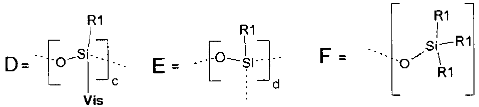 where R1 is a straight or branched alkyl, cycloalkyl, polycycloalkyl, heterocycloalkyl, alkaryl, alkoxy, aryl, aralkyl, alkenyl, alkynyl or fluorocarbon group containing 1 - 50 carbon atoms;
R2 is R1 or -H, -OH or an organic moiety containing carbon, nitrogen, phosphorous, sulphur, oxygen or silicon atoms;
"Vis" is a uv-visible absorbing moiety selected from the group consisting of flavonoids, curcuminoids, polyphenols and derivatives thereof covalently linked to a polymer chain; R3 is an organic moiety (or spacer) containing carbon, nitrogen, phosphorous, sulphur, oxygen or silicon atoms;
units A and F are the terminal moieties;
blocks B, C, D and E are non-terminal moieties independently positioned between A and F in any order;
a, c and d are integers ranging from about 1-10,000; and
b is an integer ranging from about 0-10,000.

The term "uv-visible absorbing moiety" as used herein means a moiety having a molar extinction coefficient of at least 50 units. The uv-visible absorbing moiety is preferably a curcuminoid.

It is particularly preferred that the units of block E are cross-linked with another series of units of block E through R3 to form a net like architecture.

According to a preferred aspect of the present invention there is provided a photoprotective personal care composition comprising from 0.01 to 10% by weight the silicone compound of the invention.

According to yet another aspect of the present invention there is provided use of the silicone compound of the invention as a sunscreen agent.

According to yet another aspect of the present invention there is provided use of the silicone compound of the invention as an SPF booster in a sunscreen composition comprising organic UV-A sunscreen and organic oil soluble UV-B sunscreen.

According to yet another aspect of the present invention there is provided a process for the preparation of a silicone compound of the invention comprising the steps of
(a) reacting
   (i) an Si-H containing siloxane with
   (ii) a compound of the formula

      Vi-O-(CH₂)₀₋₅₀-CH=CH₂

      or

      CH₂=CH-(CH₂)₀₋₅₀-Vi-O-(CH₂)₀₋₅₀-CH=CH₂

      or

      Vi-O-(CH₂)₀₋₅₀-C≡CH

      or

      CH₂=CH-(CH₂)₀₋₅₀-Vi-O-(CH₂)₀₋₅₀-C≡CH

      where Vi is a uv-visible absorbing moiety selected from the group consisting of flavonoids, curcumoids, and derivatives thereof polyphenols; and with
   (iii) a difunctional spacer compound having alkenyl or alkynyl groups, in the presence of a catalyst and a solvent to obtain a reaction product; and
(b) adding the reaction product to a swelling agent.

### DETAILED DESCRIPTION OF THE INVENTION

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments falling within the ambit of the claims multiply dependent upon each other irrespective of the fact that claims may be found without multiple dependency or redundancy.

### Curcuminoids

Curcuminoids include curcumins and derivatives of curcumins with different chemical groups that have been formed to increase solubility of curcumins and make them suitable for drug formulation. These compounds are polyphenols and produce a yellow color. Many curcuminoids are unsuitable for drug design because they have poor solubility in water at acidic and physiological pH, and also hydrolyze rapidly in alkaline solutions. Therefore some curcumin derivatives are synthezised to increase their solubility and hence bioavailability. Curcuminoids are soluble in dimethyl sulfoxide (DMSO), acetone and ethanol, but are poorly soluble in lipids. It is possible to increase their solubility in aqueous phase with surfactants or co-surfactants. There have been synthesized curcumin derivatives that could possibly be more potent than curcumin. Most common derivatives have different substituents on the phenyl groups.

Chemical structures of various preferred uv-visible absorbing moieties of the curcuminoid class are given below:

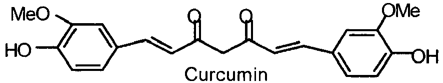

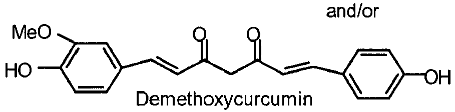

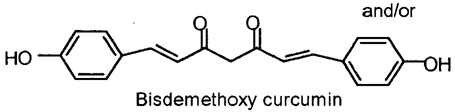

### Flavonoids

The term flavonoid (or bioflavonoid) refers to a class of plant secondary metabolites. According to the IUPAC nomenclature they can be classified into:
- flavonoids, derived from a 2-phenylchromen-4-one (2-phenyl-1,4-benzopyrone) structure 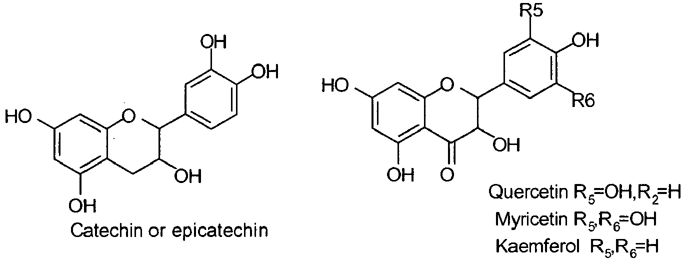
- isoflavonoids, derived from a 3-phenylchromen-4-one (3-phenyl-1,4-benzopyrone) structure 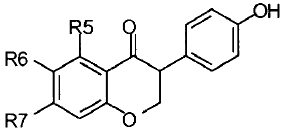 Genistein R₅=OH,R₆=H,R₇=OH
   Daidzein R₅,R₆=HR₇=OH
   Glycitein R₅=H,R₆=OCH₃,R₇=OH
   R₅=R₆=R₇=H or OH or OCH₃ and combination thereof
- neoflavonoids, derived from a 4-phenylcoumarine (4-phenyl-1,2-benzopyrone) structure.

### Polyphenols

Polyphenols are a group of chemical substances found in plants, characterized by the presence of more than one phenol unit or building block per molecule and exemplified by ellagic acid.

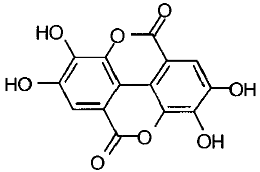

### The difunctional spacer group and R3

R3 is an organic moiety (or spacer) containing carbon, nitrogen, phosphorous, sulphur, oxygen or silicon atoms. The difunctional spacer group has two terminal alkenyl groups. The base silicone polymer chains are covalently linked to each other through difunctional spacer groups. These difunctional spacer groups prevent excessive and three dimensional cross-linking, which leads to a gelled mass. Instead they help in forming a cross-linked elastomeric compound that is relatively easier to incorporate in personal care compositions. The difunctional spacer group is preferably of the formula, 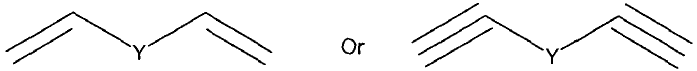 where Y is an organic moiety containing carbon, nitrogen, phosphorous, sulphur, oxygen or sllicon atoms. The difunctional spacer compound is preferably selected from the group consisting of dialkenyl polyethers, alpha omega dienes, alpha omega diynes, alpha omega ene- ynes or dialkenyl or dialkynyl terminated polysiloxane. Suitable examples of alpha omega- dienes are 1,4-pentadiene, 1,5-hexadiene, 1,7-octadiene, 1,8-nonadiene, 1,9-decadiene, 1,11-dodecadiene, 1,13-tetradecadiene and 1,19-eicosadiene. Suitable examples of alpha omega- diynes are 1,3-butadiyne or 1,5-hexadiyne, whereas the alpha omega ene-yne is preferably hexene-5-yne. It is further preferred that the spacer groups are siloxane or polyether based. Dialkenyl terminated polysiloxanes are preferably of the general formula: 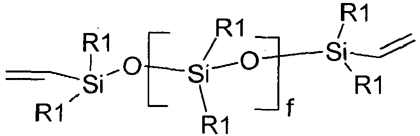 wherein R1 is a straight or branched alkyl, cycloalkyl, polycycloalkyl, heterocycloalkyl, alkaryl, alkoxy, aryl, aralkyl, alkenyl, alkynyl or fluorocarbon group containing approximately 1 to approximately 50 carbon atoms wherein f is an integer ranging from approximately 1-10,000. More preferably f is in the range of 300-500 corresponding to an average molecular weight in the range of 22,000-40,000 Daltons. A vinyl terminated polysiloxane having repeat units 375, and molecular weight of 28,000 Daltons is particularly preferred.

The dialkenyl terminated polyethers can be represented by the following general formula: 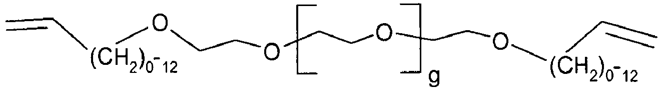 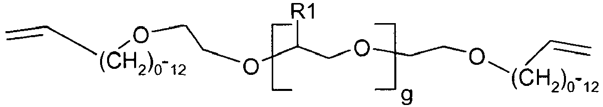 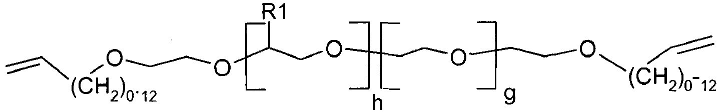 where R1 is a straight or branched alkyl, cycloalkyl, polycycloalkyl, heterocycloalkyl, alkaryl, alkoxy, aryl, aralkyl, alkenyl, alkynyl or fluorocarbon group containing approximately 1 to approximately 50 carbon atoms or -H wherein g and h are an integer ranging from approximately 1-10,000.

### Units A and F

Units A and F are the terminal moieties. In the units A and F, the functional R1 is preferably methyl or ethyl.

### Blocks B, C, D and E

Blocks B, C, D, and E are non-terminal moieties independently positioned between A and F in any order. Thus the order may be B-C-D-E as shown in the formula in the summary of the invention, or the order could be any permutation and combination which satisfies the rule "independently positioned between A and F in any order" i.e. it may be B-D-C-E, B-C-E-D, D-C-E-B and a host of other possibilities.

In the process of the invention, the Si-H containing siloxane is of the general formula: 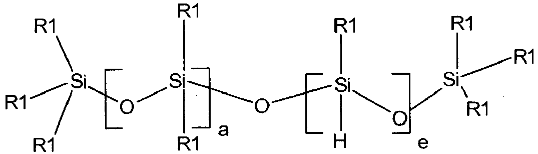 where a and e are integers ranging from about 1-10,000. The Si-H containing siloxane is preferably present at 0.001 to 95 % by weight of the reaction mixture

The compound of the formula Vi-O-(CH₂)₀₋₅₀-CH=CH₂ or CH₂=CH-(CH₂)₀₋₅₀-Vi-O-(CH₂)₀₋₅₀-CH=CH₂ or Vi-O-(CH₂)₀₋₅₀-C≡CH or CH₂=CH-(CH₂)₀₋₅₀-Vi-O-(CH₂)₀₋₅₀-C≡CH is preferably present in 0.001 to 95 % by weight of the reaction mixture (ie compounds (i), (ii), (iii), catalyst and solvent).

The difunctional spacer compound is preferably present at 0.001 to 95 % by weight of the reaction mixture.

The process is preferably carried out in the presence of a monofunctional organic moiety of the general formula 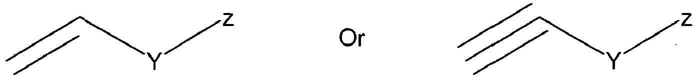 where z is -R1 or -OH or -H. The compounds most preferred for providing the functionality of block C (ie R2) are polyethylene glycol monoallyl ethers (Clarient) and long chain alkenes such as octadecene (Sigma Aldrich) having general structure indicated below: 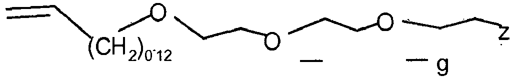 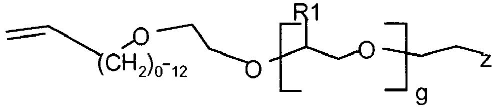 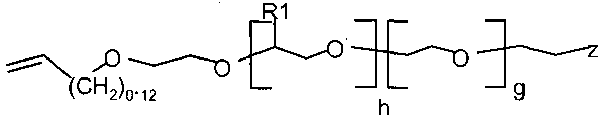 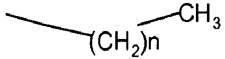 where n is an integer ranging from approximately 1-10,000, or 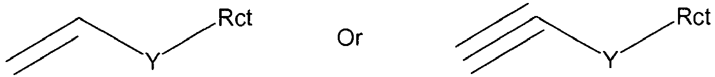 where Rct is a reactive group containing carbon, nitrogen, phosphorous, sulphur, oxygen or silicon atoms. Alternately the mono functional organic moiety of the step (a) is preferably of a general formula: 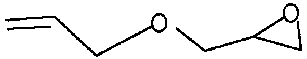 or

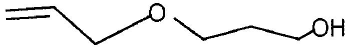

Block C has been found to be useful to include in the compound of the invention since it can be selected to provide useful properties like emulsification and act as a solubilizer for other oleophilic materials like sunscreens. The monofunctional organic moiety is preferably present in 0.001 to 95 % by weight of the reaction media.

### Catalyst

Step (a) is carried out in the presence of a catalyst. The catalyst is preferably selected from metal complexes or their compounds or metals in free or immobilized form. Transition metals such as platinum, palladium and rhodium are particularly preferred. Preferred catalysts include chloroplatinic acid, complexes of platinum with unsaturated compounds e.g. a platinum(0)-1,3-divinyl-1,1,3,3-tetramethyldisiloxane complex; platinum(0)-2,4,6,8-tetramethyl-2,4,6,8-tetravinylcyclotetrasiloxane complex; Pt(0)1,5 cyclooctadiene i.e. Pt(COD)]; platinum phospine complexes; platinum on carbon; platinum on inorganic supports such as silica and platinum black. Complexes of other metals such as palladium or rhodium may also be used for the reaction, for example, Wilkinson's catalyst RhCl [(C6H5) P]3. The catalyst can be in heterogeneous phase, eg. on charcoal or, preferably, in homogeneous phase (Karstedt catalyst). platinum(0)-1,3-divinyl-1,1,3,3-tetramethyldisiloxane complex is the most preferred catalyst. The catalyst is preferably used in an amount of 0.0001 to 20 % by weight of the reaction mixture.

### Reaction media:

The reaction is carried out in the presence of a reaction media which is a solvent which is selected from the group consisting of water, a silicone fluid, polar organic compound, a non-polar organic compound and mixtures thereof. Typically the solvent is present in an amount of 0.1 to 99.89 % by weight of the reaction mixture. Preferably the solvent is present in an amount of from 1 to 80 % by weight and more preferably from 1 to 50 % by weight of the reaction mixture. When the solvent is a polar or non-polar organic compound, it is preferred that the amount to be used is that which would create a product containing <40 % by weight solids. When used, the solvent becomes an integral part of the resulting elastomer composition and affects the structural and physical properties of the silicone elastomer. Preferably the solvent is not removed from the silicone elastomer composition. Silicone fluids useful as the solvents include alkyl and/or aryl siloxanes. Preferred are volatile methyl siloxanes (VMS). Linear VMS have the formula (CH₃)₃SiO{(CH₃)₂SiO}₀₋₅Si(CH₃)₃. Cyclic VMS have the formula {(CH₃)₂SiO}₃₋₉. Preferably the volatile methyl siloxane has a boiling point at one atmosphere of less than about 250° C. Representative linear volatile methyl siloxanes include, but are not limited to, hexamethyldisiloxane, octamethyltrisiloxane, decamethyltetrasiloxane, dodecamethylpentasiloxane, tetradecamethylhexasiloxane and hexadecamethylheptasiloxane. Representative cyclic volatile methyl siloxanes are hexamethylcyclotrisiloxane, octamethylcyclotetrasiloxane, decamethylcyclopentasiloxane and dodecamethylcyclohexasiloxane. Representative branched volatile methyl siloxanes are heptamethyl-3-{(trimethylsilyl)oxy}trisiloxane, hexamethyl-3,3-bis{(trimethylsilyl) oxy}trisiloxane and pentamethyl {(trimethylsilyl)oxy} cyclotrisiloxane. The silicone fluid useful herein also includes polysiloxane compound represented respectively by formulas (R₁)₃SiO((R₁)₂SiO)₁₋₁₀₀₀₀Si(R₁)₃ and ((R₁)₂SiO)ₚ wherein R₁ is as defined above and p is 3-9. Illustrative of such silicone fluids are polydimethylsiloxane, polydiethylsiloxane, polymethylethylsiloxane, polymethylphenylsiloxane and polydiphenylsiloxane. Organofunctional silicone fluids can also be employed as the solvent. Examples of functional silicone fluids include, but are not limited to, are acrylamide functional silicone fluids, acrylate functional silicone fluids, carbinol functional silicone fluids, carboxy functional silicone fluids, chloroalkyl functional silicone fluids, glycol functional silicone fluids, ketal functional silicone fluids, mercapto functional silicone fluids, methyl ester functional silicone fluids, perfluoro functional silicone fluids, polyisobutylene (PIB) functional silicone fluids, silanol functional silicone fluid and vinyl functional silicone fluids. When silicone fluids are used, the resulting ultraviolet radiation absorbing silicone compound is in the form of silicone gels.

Polar organic compounds useful herein include monohydroxy alcohols such as ethyl alcohol and isopropyl alcohol; diols and triols such as propylene glycol, 2-methyl-1,3-propane diol HOCH₂CH(CH₃)CH₂OH, 1,2-hexanediol CH₃(CH₂)₃CH(OH)CH₂OH, and glycerol; glycerol esters such as glyceryl triacetate (triacetin), glyceryl tripropionate (tripropionin) and glyceryl tributyrate (tributyrin); and polyglycols such as polyethylene glycols and polypropylene glycols, among which are PPG-14 butyl ether C₄H₉[OCH(CH₃)CH₂]₁₄OH.

Non-polar organic compounds may also be used as the solvents The non-polar organic compounds include aromatic hydrocarbons, aliphatic hydrocarbons, alcohols, aldehydes, ketones, amines, esters, ethers, glycols, glycol ethers, alkyl halides, or aromatic halides.

Representative compounds are alcohols such as methanol, ethanol, 1-propanol, cyclohexanol, benzyl alcohol, 2-octanol, ethylene glycol, propylene glycol and glycerol; aliphatic hydrocarbons such as pentane, cyclohexane, heptane, Varnish Maker's & Painter's (VM&P) solvent and mineral spirits; alkyl halides such as chloroform, carbon tetrachloride, perchloroethylene, ethyl chloride and chlorobenzene; aromatic hydrocarbons such as benzene, toluene, ethylbenzene and xylene; esters such as ethyl acetate, isopropyl acetate, ethyl acetoacetate, amyl acetate, isobutyl isobutyrate, benzyl acetate and isopropyl palmitate; ethers such as ethyl ether, n-butyl ether, tetrahydrofuran and 1,4-dioxane; glycol ethers such as ethylene glycol monomethyl ether, ethylene glycol monomethyl ether acetate, diethylene glycol monobutyl ether and propylene glycol monophenyl ether; ketones such as acetone, methyl ethyl ketone, cyclohexanone, diacetone alcohol, methyl amyl ketone and diisobutyl ketone; petroleum hydrocarbons such as petroleum jelly, mineral oil, gasoline, naphtha, kerosene, gas oil, heavy oil and crude oil; lubricating oils such as spindle oil and turbine oil; and fatty oils such as corn oil, soybean oil, olive oil, rape seed oil, cotton seed oil, sardine oil, herring oil and whale oil. When a polar or non-polar organic solvent is used, the resulting silicone compound is in the form of a silicone gel. Suitable organic solvents are the ones that do not undergo a chemical reaction with any of the components of the silicone phase under the anticipated conditions of processing and use and that are suitable for use in the intended end-use application.

The reaction temperature, depending upon the reactants, is in the range of 0 to 250°C and preferably about 80 - 120°C and most preferably about 110°C. The reaction time may vary between 1 minute to about 48 hours, more preferably between 1 hour to 12 hours.

### Swelling agent

After the reaction is taken to desired completion, the contents are then added to a swelling agent. The swelling agent is preferably a solvent which is used in the step (a) of the reaction with the exception of water or alcohol. Thus the swelling agent may be a silicone fluid, a polar organic compound, or a non-polar organic compound with the above exceptions. The swelling agent is most preferably a silicone fluid or a functional silicone fluid. The swelling agent is preferably used in an amount which is in a weight ratio of 1:10 to 10:1, more preferably 1:1 to 5:1 with respect to the reaction mixture of step (a).

An additional advantage of the present invention is that the silicone compound of the invention can be used as a delivery vehicle for active ingredients such as oil soluble vitamins, fragrances and sunscreens. Fragrance oils that are compatible with silicone elastomers can be absorbed into the silicone compound of the invention and their volatility will be reduced, thereby improved the desired sensorial impact.

A highly desired advantage of the present invention is that the silicone compound of the invention when incorporated in sunscreen compositions comprising well known UV-A organic sunscreens and oil-soluble UV-B sunscreens synergistically boosts the sunprotection factor (SPF). Preferred UV- A sunscreen for getting this benefit is Parsol - 1789. Preferred oil soluble UV-B sunscreen for getting this benefit is selected from the class of cinnamic acid, salicylic acid, diphenyl acrylic acid or derivatives thereof. Examples of such oil soluble organic sunscreen are Octisalate^{™}, Homosalate^{™}, NeoHelipan^{™}, bctocrylene^{™} or Parsol MCX^{™}. Most suitable such oil soluble UV-B organic sunscreen is Parsol MCX.

### Preferred reaction scheme

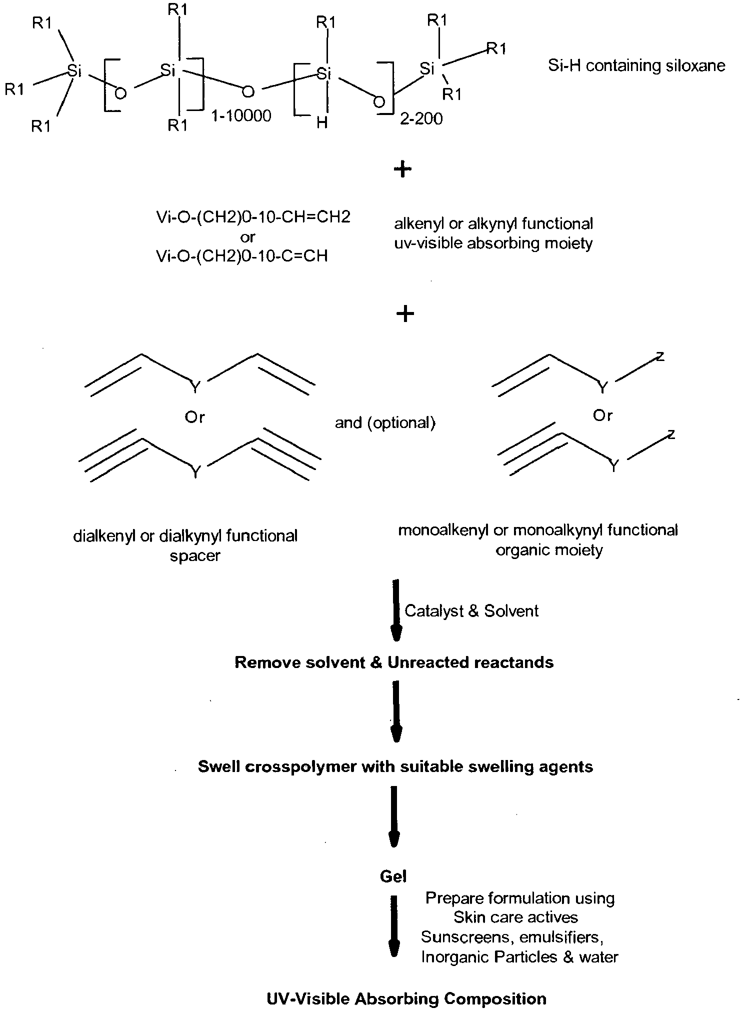

When the straight chain silicone polymer was synthesized without the use of the crosslinking spacer molecule, a product in a rubbery mass was produced. This product was difficult to swell and hence cannot be used easily in skin care formulation. The reaction scheme without the use of the crosslinking spacer molecule is shown below. 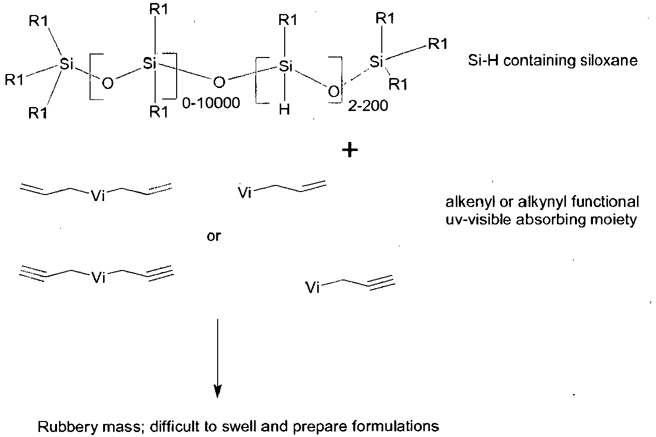 The above scheme indicates the advantage of providing the spacer molecule between the straight chain silicones to form a cross-linked product as per the present invention as compared to a straight chain silicone polymer as disclosed in EP0842938 or US2009/0209743

### Personal care composition

"Personal Care Composition" as used herein, is meant to include a composition for topical application to skin and/or hair of mammals, especially humans. Such a composition may be generally classified as leave-on or rinse off, and includes any product applied to a human body for improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of personal care compositions include leave-on skin lotions and creams, shampoos, conditioners, shower gels, toilet bars, antiperspirants, deodorants, dental products, shave creams, depilatories, lipsticks, foundations, mascara, sunless tanners and sunscreen lotions. "Skin" as used herein is meant to include skin on the face and body (e.g. neck, chest, back, arms, underarms, hands, legs, buttocks and scalp).

According to yet another aspect, the present invention relates to a personal care composition comprising the silicone compound according to the invention, in a cosmetically acceptable vehicle. It is preferred that the compound is present from 1 to 30% by weight of the composition, more preferably from 2 to 15% and most preferably from 3 to 10% by wt of the composition. These are suitable for the protection of human skin and/or hair from damaging effects of UV radiation.

The personal care compositions of the invention are useful as compositions for photo protecting the human epidermis or hair against the damaging effect of UV irradiation, as antisun/sunscreen composition or as makeup product. Such compositions can, in particular, be provided in the form of a lotion, a thickened lotion, a gel, a cream, cleansing milk, an ointment, a powder or a solid tube stick and may optionally be packaged as an aerosol and may be provided in the form of a mousse, foam or a spray.

The personal care compositions of the invention can also contain usual cosmetic adjuvants and skin care additives commonly employed in skin care products such as liquid or solid emollients, silicone oils, emulsifiers, solvents, humectants, polymeric or inorganic thickeners, powders, pigments (example clay mineral, barium sulfate, or pearl pigments, for example silver or gold, or any iris foil pearl pigment, having an interference color of red, orange, green, blue, or purple (including any iris foil pearl pigments covered with inorganic pigments, organic pigments, laked pigments, etc.), bismuth oxychloride and bismuth oxychloride coated mica), organic or inorganic sunscreens with and without photostabiliser, skin lightening agents, skin conditioners, optical brighteners, propellants, healing agents (for example allantoin), cooling agents (for example urea, menthol, menthyl lactate and frescolate), antiseptic agents and other specific skin-benefit actives, skin care actives such as skin lightening actives, anti-aging, anti-acne, anti-bacterials, antiperspirant agents, etc. The vehicle may also further include adjuncts such as antioxidants, perfumes, opacifiers, preservatives, colorants and buffers. The necessary amounts of the cosmetic and dermatological adjuvants and additives can, based on the desired product, easily be chosen by the skilled person.

The composition may additionally comprise from 0.1 % to 20%, more preferably from 0.1 % to 5 % by weight of the composition of an inorganic sunscreen agent. Inorganic sunscreens which may be employed are for eg. titanium dioxide, zinc oxide or silica such as fumed silica and mixtures thereof. These are preferably in the micronized form. Ultrafine titanium dioxide in either of its two forms, namely water-dispersible titanium dioxide and oil-dispersible titanium dioxide, may be suitable for the invention. Water-dispersible titanium dioxide is an ultra-fine titanium dioxide, the particles of which are non-coated or which are coated with a material to impart a hydrophilic surface property to the particles. Examples of such materials include aluminium oxide and aluminium silicate. Oil-dispersible titanium dioxide is an ultrafine titanium dioxide, the particles of which exhibit a hydrophobic surface property and which, for this purpose, can be coated with metal soaps such as aluminium stearate, aluminium laurate or zinc stearate, or with organosilicone compounds. By "ultrafine or micronized form" is meant particles of inorganic sunscreens having an average particle size of less than 100 µm, preferably less than 70 µm or less, more preferably less than 40 µm and most preferably from 15 µm to 25 µm.

Vitamins, which act as skin-lightening ingredients, can be advantageously included in the composition to provide for additional skin lightening effects. These include vitamin B3, vitamin B6, vitamin C, vitamin A or their precursors and cosmetically acceptable derivatives. Mixtures of the vitamins can also be employed in the composition of the invention. When present, these vitamins are used in the range of 0.01 to 10.0% by weight of said composition.

Emollients such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palpitate, silicone oils such as dimethylpolysiloxane, organomodified silicones such as cetyl dimethicone, steryl dimethicones, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate and myristyl myristate; propellants, such as propane, butane, isobutane, dimethyl ether, carbon dioxide and nitrous oxide; solvents, such as ethyl alcohol, isopropanol, acetone/ ethylene glycol monoethyl ether, diethylene glycol monobutyl ether and diethylene glycol monoethyl ether; powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose and ethylene glycol monostearate; plant extracts such as those from genus Rubia, Symplocus, Curcuma and various perfume/fragrance ingredients may also be included in the composition at ranges from 0.001 to 40.0% by weight of the composition. The emollient is preferably present in an amount from about 1 to about 20% by weight, preferably from about 2 to about 15 % by weight, and most preferably from about 4 to about 10 % by weight of the composition.

The preservatives and antioxidants are preferably present in an amount ranging from about 0.01 to about 10% by weight of the composition. Preferably the preservatives and/or antioxidants are present in an amount varying from about 0.1 to about 1% by weight of the composition.

Preferred emulsifiers that may be used to form O/W, W/O and/or O/W/O formulations include sorbitan oleate, sorbitan sesquioleate, sorbitan isostearate, sorbitan trioleate, polyglyceryl-3-diisostearate, polyglycerol esters of oleic/isostearic acid, polyglyceryl-6-hexaricinolate, polyglyceryl-4-oleate, polygylceryl-4-oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, silicone based emulsifiers and mixtures thereof.

The oily phase of the compositions according to the present invention may also contain natural vegetable or animal waxes such as bee wax, china wax, bumblebee wax and other waxes of insects as well as shea butter.

The aqueous phase of the formulations of the present invention may contain the usual cosmetic additives such as alcohols, especially lower alcohols, preferably ethanol and so or isopropanol, low alkyl diols or polyols and their ethers, preferably propyleneglycol, glycerine, ethyleneglycol, ethylene glycol monoethyl or monobutyl ether, electrolytes and especially, one or more thickeners. Thickeners that may be used in formulations of the present invention include the family of silicon dioxide, magnesium and/or aluminum silicates, polysaccharides and their derivatives such as hyaluronic acid, xanthan gum, hydroxypropyl cellulose, acrylate copolymers, preferably a polyacrylate of the family of carbopols, such as carbopols of type 980, 981, 1382, 2984 and 59S4.

Moisturizing agents, such as humectants, may be incorporated into the compositions according to the present invention to reduce the trans-epidermal water loss (TEWL) of the horny layer of the skin. Suitable humectants include glycerin, lactic acid, pyrrolidone carbonic acid, urea, polyethylene glycol, polypropylene glycol, sorbitol, PEG-400, and mixtures thereof. Additional suitable moisturizers are polymeric moisturizers of the familiy of water soluble and/or with water gelating polysaccarides such as hyaluronic acid, chitosan and/or fucose rich polysaccharides available e.g. as Fucogel 1000 (CAS-Nr. is 178463-23-5) from Solabia. The moisturizing agent is optionally present in an amount varying from about 0.5 to about 8 % by weight of the composition, preferably from about 1 to about 5 % by weight of the composition.

Suitable neutralizing agents which may be included in the composition of the present invention to neutralize components such as e.g. an emulsifier or a foam builder/stabilizer include but are not limited to alkali hydroxides such as a sodium and potassium hydroxide, organic bases such as diethanolamine (DEA), triethanolamine (TEA), aminomethyl propanol, trisodium ethylenediaminetetraacetic acid and mixtures thereof; basic amino acids such as arginine and lysine and any combination of any of the foregoing. The neutralizing agent may be present in an amount of about 0.01 to about 8 % by weight of the composition, preferably 1 to about 5% by weight of the composition. The addition of electrolytes into the composition of the present invention may be necessary to change the behavior of a hydrophobic emulsifier. Thus the emulsions may preferably contain electrolytes of one or several salts including anions such as a chloride, a sulfate, a carbonate, a borate or an aluminate, without being limited thereto. Other suitable electrolytes may be on the bases of organic anions such as, but not limited to, lactate, acetate, benzoate, propionate, tartrate and citrate. As cations preferred are ammonium, alkyl ammonium, alkaline or alkaline earth metals such as sodium or magnesium. Especially preferred salts are potassium and sodium chloride, magnesium sulfate, zinc sulfate and mixtures thereof. Electrolytes are preferably present in an amount of about 0.01 to about 0.5 % by weight of the composition.

The invention will now be explained in detail with help of the following non-limiting examples, which form preferred embodiments of the various aspects of the invention.

### Example 1: Synthesis of UV -visible light absorbing silicone compound having a curcumin moiety

This silicone compound was synthesized in four stages:
Stage 1: Synthesis of allyloxy functional curcumin derivative (a compound of the formula Vi-O-(CH₂)₀₋₅₀-CH=CH₂ or Vi-O-(CH₂)₀₋₅₀-C≡CH in step (a) (ii) of the process of the invention)
   A 2-liter, 3-neck flask was fitted with a condenser and funnel. The flask was charged with 5g (0.014mol) of curcumin and 17.7 ml of ethanol. Sodium ethoxide 2.268 g (0.042 mol) as a 21% solution in ethanol was added rapidly. The mixture was heated to 50-60 °C for 2 hours. Allyl bromide (3.7 g.(0.042 mol)) was added and the mixture heated to reflux
   (70 - 74 °C) for 20 hours. The mixture was allowed to return to room temperature and 50 ml water were added, followed by 17 ml of toluene and about 1 ml of 38% aqueous hydrochloric acid. The organic layer was separated and washed with 50 ml of water. Volatiles were removed from the organic layer by rotary evaporation at 80 °C and at 2 mm Hg to give 5 g of allyl ether intermediate. The intermediate consisted primarily of 1,7 bis(4-allyloxy-3-methoxyphenyl)-1,6-heptadiene-3,5-dione. A dark brownish yellow coloured sticky solid was obtained having ¹H-NMR (CDCl₃) peaks (due to allyl and methoxy group present on curcumin molecule) at δ 6.05 (CH₂=CH-CH₂-O-), 5.32 & 5.43 (CH₂=CH-CH₂-O), 4.6 (CH=CH₂-CH₂-O), 3.9 (CH₃O-).
Stage 2: Synthesis of methylhydrogenpolysiloxane (MHPS) copolymer (Si-H containing siloxane of step (a) (i) of the process of the invention).
   50 g of octamethycyclotetrasiloxane (D₄) was mixed with 15 g of methylhydrogenpolysiloxane (Aldrich, MHPS) in a two necked round bottom flask. To the mixture 1 g of Tulsion catalyst (Thermax, T63MP) was added. The reaction mixture was stirred at 120 °C for 4 hours. Viscous MHPS copolymer obtained was cooled down to room temperature. Catalyst was filtered off. Unreacted D₄ was distilled off under vacuum at 125 °C. The product obtained was a colourless, viscous oily substance. The colourless, viscous liquid was characterised with an FTIR peak due to Si-H at 2115 cm⁻¹, Si-CH₃ at 1260 cm⁻¹ and -Si-O-Si at 1186 cm⁻¹; and ¹H-NMR (CDCl₃) peaks at δ 0.09 (SiCH₃), 0.17 (Si(CH₃)H) and 4.68 (Si-H ). 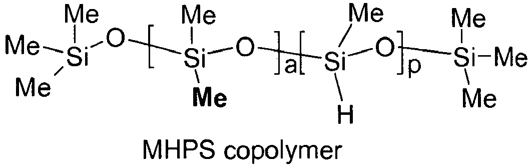
Stage 3: Synthesis of divinyl terminated polysiloxane copolymer (VTP) spacer (a difunctional spacer compound having alkenyl or alkynyl groups of step (a) (iii) of the process of the invention).
   20 g of octamethycyclotetrasiloxane (D₄) was mixed with 4 g of divinyltetramethy-disiloxane (Aldrich) in a 50 mL two necked flask. To the mixture 0.3 g of Tulsion catalyst (Thermax, T63MP) was added. The reaction mixture was stirred at 120 °C for 4 hours. Divinyl terminated polysiloxane copolymer (VTP) copolymer obtained was cooled down to room temperature. Catalyst was filtered off. Unreacted D₄ was distilled off under vacuum at 125 °C. The product obtained was a colourless, viscous oil. This was used as the spacer compound. The colourless, viscous liquid was characterised with ¹H-NMR (CDCl₃) peaks at δ 0.09 (SiCH₃), 0.17 (Si(CH₃)H), 0.8 (Si-CH=CH₂), 5.6 - 6.2 (Si-CH=CH₂). 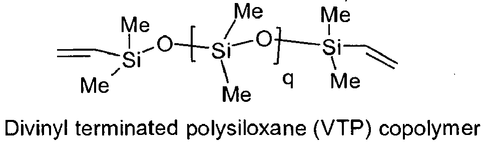
Stage 4: Hydrosilylation and swelling (step (b) of the process of the invention).
   100 mL toluene and 1.5 g of polyethylene glycol monoallyl ethers (Polyglykol A 20-10) were charged to a three necked flask fitted with a Dean and Stark set-up under nitrogen atmosphere. Traces of water present in toluene were removed by azeotropic distillation. 1.5 g allyloxy functional curcumin class (as prepared in stage 1 above); and 0.21 g divinyl terminated polysiloxane (VTP) (as prepared in stage 3 above) were charged into a moisture-free three necked flask, fitted with a reflux assembly and maintained under nitrogen atmosphere. 50 ml dry toluene was added subsequently to dissolve all the reagents. Three drops of platinum catalyst (1,3-divinyltetramethyldisiloxane, Sigma-Aldrich) were added to the reaction mixture and the mixture was stirred at room temperature for about 0.5 hours. 2 g of methylhydropolysiloxane (MHPS) copolymer (as prepared in stage 2 above) was added and the reaction stirred at about 110 °C for about 2-3 hrs. The progress of the reaction was monitored by TLC and FT-IR. The product was obtained in a gel form. The gel was further washed with methanol to remove unreacted organic matter and platinum catalyst. It was further swollen in 30 g of decamethylcyclopentasiloxane (D₅) and remaining traces of toluene and methanol were removed under vacuum below 60°C.

The absorbance of a 400 ppm solution of the product obtained in example 1 showed 2 UV absorption maxima, one at 310 nm with an absorbance value of 0.65 units and the other at 355 nm with a corresponding absorbance value of 0.80 and at 440 nm with a corresponding absorbance value of 0.26 units, thereby confirming the presence of both the UV as well as visible absorbing moieties. Thus it can be readily seen that the compound of the present invention provides protection against both UV and visible radiation as indicated by absorbance in UVA, UVB and visible radiation wavelengths.

### Example 1A: Admixture of polymer and curcumin

Curcumin (at 0.13 % by weight of the composition) was mixed with DC 9040 silicone dimethicone crosspolymer (Dow Corning®). The resultant composition was opaque and not homogeneous as particles of curcumin could be seen to be physically dispersed in the gel matrix when observed under microscope. On the other hand, the appearance of the product of example 1 where curcumin is covalently attached to silicone backbone was transparent and homogeneous and no particles could be seen when observed under microscope. Thus the composition of example 1A does not provide for uniform distribution of curcumin in the polymer thereby affording poor UV - visible radiation protection.

### Example 2: Personal care composition comprising the silicone compound of the invention

A personal care composition was prepared using the silicone compound of the invention. First the ingredients were prepared in two different phases i.e (i) an oil phase and (ii) a water (aqueous) phase. The ingredients in the respective phases are tabulated below in table 1.

**Table 1**

| Ingredients | **% by weight in the composition** |
|---|---|
| **Phase 1 (oil phase)** | |
| Product prepared in example 1 | 50 |
| Emulsifier DC5225 (Dow Corning®) | 20 |
| Titanium dioxide | 0.9 |

| **Phase 2 (aqueous phase)** | |
|---|---|
| Water | To 100 |

Ingredients from phase 1 were mixed at 25°C and homogenized for 10 minutes. Phase 2 was prepared separately at 25°C. The aqueous phase was added slowly to the oil phase until the mixture attained a homogenized condition. After homogenization, a smooth cream with acceptable spreading characteristics was obtained.

### Example 3: Commercial personal care composition

Example 3 was a commercial skin lightening cream Fair & Lovely Multivitamin containing 0.45% Parsol 1789 (UVA filter), 0.75% Parsol MCX (UVB filter) and 0.9% TiO₂.

The product of example 1 and compositions of example 2 and example 3 were applied at 3mg/cm² on a transpore tape (3M) which was fixed to a quartz plate and subjected to 5.5 mW/cm² under a simulated solar Atlas sun lamp. The application was performed using a standard Colipa procedure. The quartz plate was dried and kept in a solar simulator. The incident energy of light was adjusted to 5.5 mw/cm². The transmitted energy was measured through the film present on the transpore tape/quartz, using a radiometer. The transmitted energy obtained from the transpore tape/quartz was used as the control. The data on the % transmittance is given in table 2 below.

**Table 2**

| Examples | % Transmittance | | |
|---|---|---|---|
| | 310 nm | 360 nm | 410 nm |
| Example 1 | 23 | 15 | 36 |
| Example 2 | 41 | 35 | 57 |
| Example 3 | 24 | 35 | 91 |

Example 2 is a formulation prepared from a silicone compound of the invention, which does not contain any commercial UVA and UVB filters. On the other hand example 3 contains commercial UVA and UVB sunscreens along with inorganic particles, which could influence UVA and UVB region; but not visible region. The efficacy of example 2 was therefore slightly inferior in the UV-B region, comparable in the UV-A region, and superior in the visible region, although cream of example 2 does not contain any commercial UV sunscreens. The superiority of example as per the invention specifically brings out the protection in the visible region, which is object of the invention, with little or no compromise on the UV protection.

The data in table 2 indicates that the silicone compound as per the invention and a personal care composition prepared using the silicone compound provide for better and broader spectrum photoprotection (to include good visible protection) in comparison to a commercial sample.

### Examples 4 and 5: Improved sunprotection factor (SPF) through use of compound of the present invention

Sunscreen compositions as shown in table 3 were prepared. In-vitro SPF of these compositions were measured using an Optometrics 290S instrument model. The substrate used was a 8 cm Transpore tape procured from 3M Company. The sample was applied at 2 mg/cm². The data is summarised in table 3.

**Table 3**

| Ingredients | Example 4, wt% | Example 5, wt% |
|---|---|---|
| Sample of example 1 | 50 | - |
| Sample of example 1A | - | 50 |
| 5225C Emulsifier | 30 | 30 |
| Parsol MCX | 0.75 | 0.75 |
| Parsol 1789 | 0.4 | 0.4 |
| Water | To 100 | To 100 |
| SPF | 12 | 2.5 |

The data in table 3 indicates that the compound as per the invention when included in a sunscreen composition comprising conventional organic UV-A and UV-B sunscreens (example 4) provides for vastly improved SPF as compared to a composition where equivalent amount of curcumin is added as an admixture in the composition (example 5).

### Example 6: Hair Gel for Photoprotective benefits

Good photoprotective hair gel composition (as shown in table 4) was prepared using the silicone compound of the invention. The process comprised the steps of first mixing the compound of example 1 with Aristoflex AVC (which is a cationic polymer). The mixture was homogenized for five minutes. Parsol MCX, and a hair colorant were added subsequently. These were then mixed in a high speed homogenizer for 15 minutes. Perfume was added subsequently. This composition had good consumer accepted sensory properties. The composition is given in table 4 below:

**Table 4**

| Ingredients | wt% |
|---|---|
| Silicone compound as per example 1 | 5 |
| Aristoflex AVC ( cationic polymer) | 1.2 |
| Parsol MCX | 2.25 |
| Hair Colorant | 5 |
| Perfume | 0.3 |
| Water | To 100 |

### Example 7: Deodourant composition

A deodourant composition as shown in table 5, using the silicone compound of the invention, in the form of a cream, was prepared. The process used was to first mix the compound of example 1 with a silicone emulsifier (DC 5225C from Dow Corning Corp., Midland, Mich.). The mixture was homogenized for five minutes. Antiperspirant active (Aluminium chlorohydrate) was then added and the mixture subjected to high speed homogenization for 15 minutes. Perfume was added subsequently.

**Table 5**

| Ingredients | Wt% |
|---|---|
| Silicone compound as per example 1 | 25 |
| 5225C emulsifier | 10 |
| Antiperspirant active | 10 |
| Perfume | 0.3 |
| Water | To 100 |

## Claims

1. A silicone compound of the general formula: 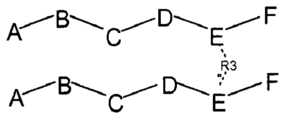 or 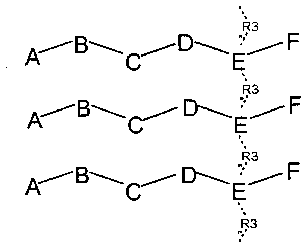 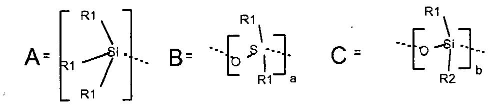 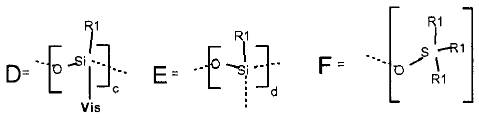 where R1 is a straight or branched alkyl, cycloalkyl, polycycloalkyl, heterocycloalkyl, alkaryl, alkoxy, aryl, aralkyl, alkenyl, alkynyl or fluorocarbon group containing 1 - 50 carbon atoms;
R2 is R1 or -H, -OH or an organic moiety containing carbon, nitrogen, phosphorous, sulphur, oxygen or silicon atoms;
"Vis" is a uv-visible absorbing moiety selected from the group consisting of flavonoids, curcuminoids, polyphenols and derivatives thereof covalently linked to a polymer chain;
R3 is an organic moiety (or spacer) containing carbon, nitrogen, phosphorous, sulphur, oxygen or silicon atoms;
units A and F are the terminal moieties;
blocks B, C, D and E are non-terminal moieties independently positioned between A and F in any order;
a, c and d are integers ranging from 1-10,000; and
b is an integer ranging from 0-10,000.

2. A silicone compound as claimed in claim 1 wherein said Vis is a curcuminoid derivative.

3. A process for the preparation of a silicone compound as claimed in claim 1 or claim 2, the process comprising the steps of:
(a) reacting
(i) an Si-H containing siloxane with
(ii) a compound of the formula
Vi-O-(CH₂)₀₋₅₀-CH=CH₂
or
CH₂=CH-(CH₂)₀₋₅₀-Vi-O-(CH₂)₀₋₅₀-CH=CH₂
or
Vi-O-(CH₂)₀₋₅₀-C≡CH
or
CH₂=CH-(CH₂)₀₋₅₀-Vi-O-(CH₂)₀₋₅₀-C≡CH
where Vi is a uv-visible absorbing moiety selected from the group consisting of flavonoids, curcumoids, polyphenols and derivatives thereof; and with
(iii) a difunctional spacer compound having alkenyl or alkynyl groups, in the presence of a catalyst and a solvent to obtain a reaction product, and
(b) adding the reaction product to a swelling agent.

4. A process as claimed in claim 3 wherein the difunctional spacer group is of the formula 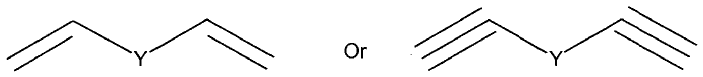 where Y is an organic moiety containing carbon, nitrogen, phosphorous, sulphur, oxygen or silicon atoms.

5. A process as claimed in claim 4 wherein Y is selected from a group consisting of hydrocarbons, polysiloxanes, polyethers, polycarboxylic acid and polysaccharides.

6. A process as claimed in any one of claims 3 to 5 wherein the difunctional spacer compound is selected from the group consisting of dialkenyl polyethers, alpha omega dienes, alpha omega diynes; alpha omega ene- ynes or dialkenyl or dialkynyl terminated polysiloxane.

7. A process as claimed in any one of claims 3 to 6 wherein the Si-H containing siloxane is of the general formula: 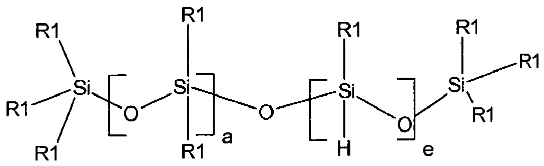 where a and e are integers ranging from about 1-10,000.

8. A process as claimed in any one of claims 3 to 7 wherein step (a) is carried out in the presence of a monofunctional organic moiety of the general formula 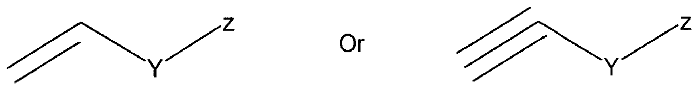 where z is -R1 or -OH or -H or 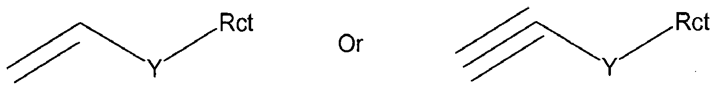 where Rct is a reactive group containing carbon, nitrogen, phosphorous, sulphur, oxygen or silicon atoms.

9. A process as claimed in any one of claims 3 to 8 wherein said solvent is selected from the group consisting of water, a silicone fluid, polar organic compound, a non-polar organic compound and a mixture thereof.

10. A process as claimed in any one of claims 3 to 9 wherein said swelling agent is a solvent as claimed in claim 9 with the exception of water or alcohol.

11. A process as claimed in claim 10 wherein said swelling agent is a silicone fluid or a functional silicone fluid.

12. A photoprotective personal care composition comprising from 0.01 to 10% by weight of the silicone compound as claimed in claim 1.

13. Use of a silicone compound as claimed in claim 1 as a sunscreen agent.

14. Use of a silicone compound as claimed in claim 1 as an SPF booster in a sunscreen composition comprising organic UV-A sunscreen and organic oil soluble UV-B sunscreen.

## Patentansprüche

1. Siliconverbindung der allgemeinen Formel: 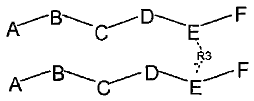 oder 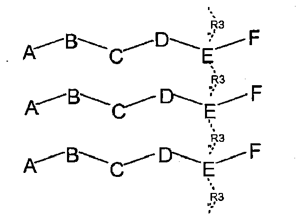 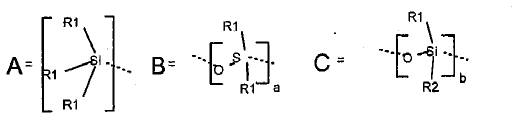 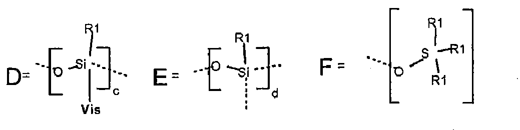 worin:
R1 ein geradkettiger oder verzweigter Alkyl-, Cycloalkyl- Polycycloalkyl-, Heterocycloalkyl-, Alkaryl-, Alkoxy-, Aryl-, Aralkyl-, Alkenyl-, Alkinyl- oder
Fluorkohlenstoffrest mit 1 bis 50 Kohlenstoffatomen ist;
R2 gleich R1 oder -H, -OH oder eine organische Einheit ist, die Kohlenstoff-, Stickstoff-, Phosphor-, Schwefel-, Sauerstoff- oder Siliciumatome enthält;
"Vis" eine UV-VIS absorbierende Einheit ist, die aus der Gruppe ausgewählt ist,
die aus Flavonoiden, Curcuminoiden, Polyphenolen und Derivaten davon besteht,
die kovalent an eine Polymerkette gebunden ist;
R3 eine organische Einheit (oder ein Spacer) ist, die Kohlenstoff-, Stickstoff-, Phosphor-, Schwefel-, Sauerstoff- oder Siliciumatome enthält;
die Einheiten A und F die endständigen Einheiten sind;
die Blöcke B, C, D und E keine endständigen Einheiten sind, die unabhängig voneinander in irgendeiner Reihenfolge zwischen A und F angeordnet sind;
a, c und d ganze Zahlen im Bereich von 1 bis 10000 sind; und
b eine ganze Zahl im Bereich von 0 bis 10000 ist.

2. Siliconverbindung nach Anspruch 1,
wobei Vis ein Curcuminoid-Derivat ist.

3. Verfahren zum Herstellen einer Siliconverbindung nach Anspruch 1 oder 2, wobei das Verfahren die folgenden Schritte aufweist:
(a) Umsetzen von
(i) einem Si-H enthaltenden Siloxan mit
(ii) einer Verbindung der Formel
Vi-O-(CH₂)₀₋₅₀-CH=CH₂
oder
CH₂=CH-(CH₂)₀₋₅₀-Vi-O-(CH₂)₀₋₅₀-CH=CH₂
oder
Vi-O-(CH₂)₀₋₅₀-C→CH
oder
CH₂=CH-(CH₂)₀₋₅₀-Vi-O-(CH₂)₀₋₅₀-C→CH wobei Vi eine UV-VIS absorbierende Einheit ist, die aus der Gruppe ausgewählt ist, die aus Flavonoiden, Curcumoiden, Polyphenolen und Derivaten davon besteht;
und mit
(iii) einer difunktionellen Spacerverbindung mit Alkenyl- oder Alkinylgruppen in Gegenwart eines Katalysators und eines Lösungsmittels, um ein Reaktionsprodukt zu erhalten, und
(b) Zugeben des Reaktionsproduktes zu einem Quellmittel.

4. Verfahren nach Anspruch 3,
wobei die difunktionelle Spacergruppe die Formel 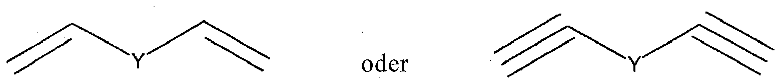 hat, worin Y eine organische Einheit ist, die Kohlenstoff-, Stickstoff-, Phosphor-, Schwefel-, Sauerstoff- oder Siliciumatome enthält.

5. Verfahren nach Anspruch 4,
wobei Y aus der Gruppe ausgewählt ist, die aus Kohlenwasserstoffen, Polysiloxanen, Polyethern, Polycarbonsäure und Polysacchariden besteht.

6. Verfahren nach einem der Ansprüche 3 bis 5,
wobei die difunktionelle Spacerverbindung aus der Gruppe ausgewählt ist, die aus Dialkenylpolyethern, α,▷-Dienen, α,▷-Diinen, α,▷-En-inen oder mit Dialkenyl oder Dialkinyl terminiertem Polysiloxan besteht.

7. Verfahren nach einem der Ansprüche 3 bis 6,
wobei das Si-H enthaltende Siloxan die allgemeine Formel 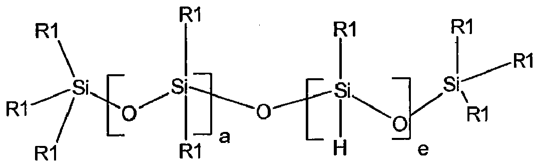 hat, worin A und E ganze Zahlen im Bereich von etwa 1 bis 10000 sind.

8. Verfahren nach einem der Ansprüche 3 bis 7,
wobei der Schritt (a) in Gegenwart einer monofunktionellen organischen Einheit der folgenden allgemeinen Formel durchgeführt wird: 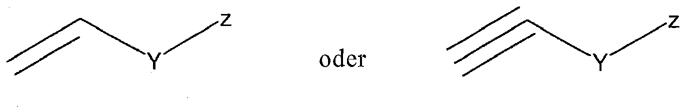 worin Z gleich -R1 oder -OH oder -H ist, 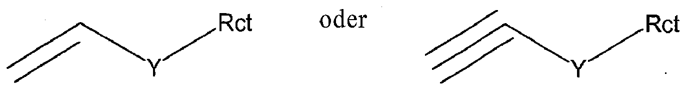 worin Rct eine reaktive Gruppe ist, die Kohlenstoff-, Stickstoff-, Phosphor-, Schwefel-, Sauerstoff- oder Siliciumatome enthält.

9. Verfahren nach einem der Ansprüche 3 bis 8,
wobei das Lösungsmittel aus der Gruppe ausgewählt ist, die aus Wasser, einem Siliconfluid, einer polaren organischen Verbindung, einer nichtpolaren organischen Verbindung und einem Gemisch davon besteht.

10. Verfahren nach einem der Ansprüche 3 bis 9,
wobei das Quellmittel ein Lösungsmittel nach Anspruch 9 ist, wobei Wasser oder Alkohol ausgenommen sind.

11. Verfahren nach Anspruch 10,
wobei das Quellmittel ein Siliconfluid oder ein funktionelles Siliconfluid ist.

12. Vor Licht schützende Körperpflegezusammensetzung,
die 0,01 bis 10 Gew.-% der Siliconverbindung nach Anspruch 1 aufweist.

13. Verwendung einer Siliconverbindung nach Anspruch 1 als Sonnenschutzmittel.

14. Verwendung einer Siliconverbindung nach Anspruch 1 als SPF-Verstärker in einer Sonnenschutzzusammensetzung, die einen organischen UV-A-Sonnenschutz und einen organischen öllöslichen UV-B-Sonnenschutz aufweist.

## Revendications

1. Composé de silicone de la formule générale : 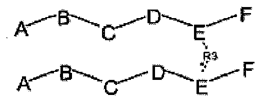 ou 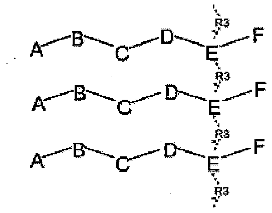 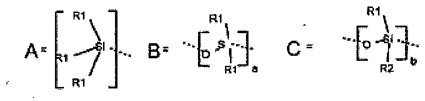 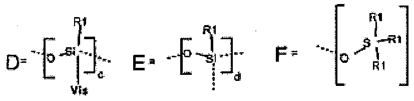 où R1 est un groupe alkyle, cycloalkyle, polycycloalkyle, hétérocycloalkyle, alkaryle, alcoxy, aryle, aralkyle, alcényle, alcynyle ou fluorocarboné linéaire ou ramifié contenant 1 - 50 atomes de carbone ;
R2 est R1 ou -H, -OH ou une moitié organique contenant des atomes de carbone, d'azote, de phosphore, de soufre, d'oxygène ou de silicium ;
"Vis" est une moitié absorbant les UV visibles choisie dans le groupe constitué de flavonoïdes, de curcuminoïdes, de polyphénols et de dérivés de ceux-ci liés de manière covalente à une chaîne polymère ;
R3 est une moitié organique (ou écarteur) contenant des atomes de carbone, d'azote, de phosphore, de soufre, d'oxygène ou de silicium ;
les unités A et F sont les moitiés terminales ;
les séquences B, C, D et E sont des moitiés non-terminales indépendamment positionnées entre A et F dans un ordre quelconque ;
a, c et d sont des nombres entiers de 1- 10 000 ; et
b est un nombre entier de 0 - 10 000.

2. Composé de silicone selon la revendication 1, dans lequel ledit Vis est un dérivé de curcuminoïde.

3. Procédé pour la préparation d'un composé de silicone selon la revendication 1 ou la revendication 2, le procédé comprenant les étapes :
(a) de réaction
(i) d'un siloxane contenant Si-H avec
(ii) un composé de la formule
Vi-O-(CH₂)₀₋₅₀-CH=CH₂
ou
CH₂=CH-(CH₂)₀₋₅₀-Vi-O-(CH₂)₀₋₅₀-CH=CH₂
ou
Vi-O-(CH₂)₀₋₅₀-C≡CH
ou
CH₂=CH-(CH₂)₀₋₅₀-Vi-O-(CH₂)₀₋₅₀-C≡CH
où Vi est une moitié absorbant les UV visibles choisie dans le groupe constitué de flavonoïdes, de curcumoïdes, de polyphénols et de dérivés de ceux-ci ; et avec
(iii) un composé écarteur difonctionnel présentant des groupes alcényle ou alcynyle, en présence d'un catalyseur et d'un solvant pour obtenir un produit de réaction, et
(b) d'addition du produit de réaction à un agent gonflant.

4. Procédé selon la revendication 3, dans lequel le groupe écarteur difonctionnel est de la formule 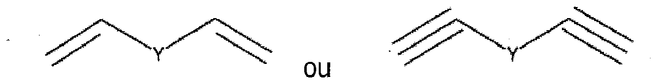 où Y est une moitié organique contenant des atomes du carbone, d'azote, de phosphore, de soufre, d'oxygène ou de silicium.

5. Procédé selon la revendication 4, dans lequel Y est choisi dans un groupe constitué d'hydrocarbures, de polysiloxanes, de polyéthers, de poly(acide carboxylique) ou de polysaccharides.

6. Procédé selon l'une quelconque des revendications 3 à 5, dans lequel le composé écarteur difonctionnel est choisi dans le groupe constitué de dialcénylpolyéthers, de diènes alpha oméga, de diynes alpha oméga ; d'ène-ynes alpha oméga ou de polysiloxanes dialcényle ou dialcynyle terminés.

7. Procédé selon l'une quelconque des revendications 3 à 6, dans lequel le siloxane contenant Si-H est de la formule générale : 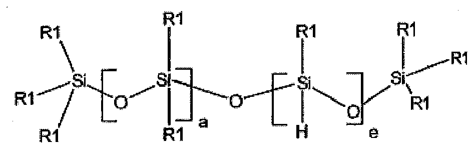 où a et e sont des nombres entiers d'environ 1-10 000.

8. Procédé selon l'une quelconque des revendications 3 à 7, dans lequel l'étape (a) est réalisée en présence d'une moitié organique monofonctionnelle de la formule générale 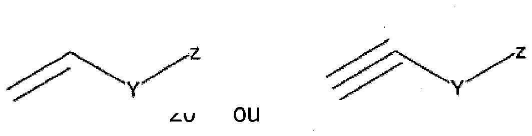 où z est -R1 ou -OH ou -H
ou 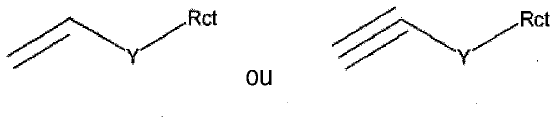 où Rct est un groupe réactif contenant des atomes de carbone, d'azote, de phosphore, de soufre, d'oxygène ou de silicium.

9. Procédé selon l'une quelconque des revendications 3 à 8, dans lequel ledit solvant est choisi dans le groupe constitué d'eau, d'un fluide de silicone, d'un composé organique polaire, d'un composé organique non-polaire et d'un mélange de ceux-ci.

10. Procédé selon l'une quelconque des revendications 3 à 9, dans lequel ledit agent gonflant est un solvant selon la revendication 9 à l'exception de l'eau ou d'un alcool.

11. Procédé selon la revendication 10, dans lequel ledit agent gonflant est un fluide de silicone ou un fluide de silicone fonctionnel.

12. Composition photoprotectrice pour l'hygiène personnelle comprenant de 0,01 à 10 % en poids du composé de silicone selon la revendication 1.

13. Utilisation d'un composé de silicone selon la revendication 1 comme un agent d'écran solaire.

14. Utilisation d'un composé de silicone selon la revendication 1 comme un renforçateur SPF dans une composition d'écran solaire comprenant un écran solaire aux UV-A organique et un écran solaire aux UV-B soluble dans une huile organique.
